Europäisches Patentamt

European Patent Office

Office européen des brevets

(19)

(11) Numéro de publication: **0 228 938**
**B1**

(12) # FASCICULE DE BREVET EUROPEEN

(45) Date de publication du fascicule du brevet:
**08.03.89**

(51) Int. Cl.⁴: **C07C 148/00**, C07C 149/247

(21) Numéro de dépôt: **86402667.9**

(22) Date de dépôt: **02.12.86**

(54) Procédé de préparation d'une solution aqueuse d'un sel alcalin de la méthionine.

(30) Priorité: **03.12.85 FR 8517847**

(43) Date de publication de la demande:
**15.07.87 Bulletin 87/29**

(45) Mention de la délivrance du brevet:
**08.03.89 Bulletin 89/10**

(84) Etats contractants désignés:
**AT BE CH DE ES FR GB IT LI LU NL SE**

(56) Documents cités:
**EP-A- 0 067 499**

(73) Titulaire: **RHONE-POULENC NUTRITION ANIMALE, Rue Marcel Lingot, F-03600 Commentry(FR)**

(72) Inventeur: **Gillonnier, Claude, Les Chaumes, F-03310 Néris-les-Bains(FR)**
Inventeur: **Moisson, René, 62, rue Saint-Jean, F-03100 Montluçon(FR)**

(74) Mandataire: **Pilard, Jacques et al, RHONE-POULENC RECHERCHES Service Brevets Pharma 25, Quai Paul Doumer, F-92408 Courbevoie Cedex(FR)**

ACTORUM AG

## Description

La présente invention concerne un procédé de préparation d'une solution aqueuse d'un sel alcalin de la méthionine utilisable comme additif dans les aliments pour les animaux d'élevage.

La méthionine, qui est un aminoacide essentiel, est utilisée à des doses faibles, généralement inférieures à 1% comme additif dans les aliments pour animaux. Il est particulièrement important de pouvoir réaliser une dispersion homogène de la méthionine dans la nourriture et de pouvoir doser la méthionine avec précision.

L'utilisation de la méthionine sous forme solide cristallisée présente des inconvénients qui rendent sa manipulation et son dosage difficiles.

L'utilisation de produits liquides permet de réaliser une dispersion homogène à des concentrations facilement contrôlables.

La méthionine étant peu soluble dans l'eau, elle ne peut pas être utilisée telle quelle en solution aqueuse du fait des volumes considérables qu'il serait nécessaire de manipuler. Toutefois, les sels de métaux alcalins de la méthionine, dont la solubilité dans l'eau est plus importante permettent d'obtenir des solutions suffisamment concentrées qui conviennent particulièrement bien pour la réalisation du but recherché.

Des solutions de méthioninate alcalin, en particulier de méthioninate de sodium, peuvent être préparées par dissolution de la méthionine dans une solution aqueuse d'un hydroxyde alcalin. Cependant, cette façon de procéder n'est pas économiquement avantageuse du fait qu'il faut préalablement isoler la méthionine avant de la dissoudre dans un milieu approprié.

Il est également connu, d'après les demandes de brevet français FR 2 499 560, FR 2 499 563, FR 2 499 564, FR 2 499 565 et FR 2 499 566 de préparer des solutions aqueuses de méthioninate de sodium par saponification de la (β-méthylmercaptoéthyl)-5 hydantoïne au moyen d'un excès de soude et/ou carbonate de sodium et/ou de chaux. Cependant, au cours de la saponification, il se forme des quantités importantes de carbonate de sodium et/ou calcium qu'il est nécessaire d'éliminer par des traitement appropriés (concentration, précipitation, filtration, ...).

D'après le brevet français FR 2 372 797, il est connu de préparer la méthionine à partir de l'aminonitrile correspondant par action d'ions hydroxydes en présence d'une cétone en passant intermédiairement par l'aminoamide. Selon ce procédé, l'hydrolyse est effectuée dans un milieu aqueux contenant une quantité d'hydroxyde de métal alcalin voisine de la stœchiométrie.

Il a maintenant été trouvé que l'hydrolyse du méthylmercapto-4 amino-2 butyramide intermédiairement obtenu peut être effectuée sans observer la formation de produits secondaires indésirables tels que des dipeptides et qu'il est ainsi possible de préparer des solutions de méthioninate alcalin, directement utilisables en alimentation animale, dont la concentration exprimée en équivalent méthionine, est comprise entre 30 et 60% et de préférence entre 40 et 45% en poids, exemptes de sous-produits néfastes et ne contenant pas de sels minéraux.

Le procédé selon l'invention consiste à chauffer dans un autoclave le méthylmercapto-4 amino-2 butyramide, éventuellement préparé in situ, selon le procédé décrit dans le brevet français FR 2 372 797, dans une solution aqueuse d'un hydroxyde alcalin à une température comprise entre 100 et 200°C pendant 5 à 10 minutes, puis, après dégazage pour éliminer l'ammoniac formé, à refroidir la solution obtenue qui est directement utilisable comme additif pour les aliments.

Généralement on utilise une quantité d'hydroxyde alcalin comprise entre 0,9 et 1,1 mole par mole d'amide mis en œuvre. Comme hydroxyde alcalin, on utilise préférentiellement la soude.

L'hydrolyse de l'amide peut être effectuée en milieu dilué, la solution de méthioninate alcalin obtenue étant amenée à la concentration désirée comprise entre 30 et 60%, de préférence entre 40 et 45%, par élimination d'eau. De préférence, l'hydrolyse est effectuée de telle manière que l'on obtienne directement la solution de méthioninate à la concentration désirée, ce qui permet d'éviter les opérations couteuses de concentration de solutions.

Les solutions de méthioninate alcalin, de préférence de méthioninate de sodium, obtenues selon le procédé de la présente invention sont stables en particulier jusqu'à des températures pouvant atteindre de −15 à −30°C selon leur concentration. Par ailleurs, les propriétés biologiques des solutions obtenues sont équivalentes à celles de la méthionine solide.

Les exemples suivants, donnés à titre non limitatif, montrent comment l'invention peut être mise en pratique.

## EXEMPLE 1

Dans un réacteur de 500 cm³ permettant de travailler sous pression on introduit 1 mole d'aldéhyde méthylmercapto-3 propionique (AMMP) et de 20 mg de triéthylamine.

On introduit 1,05 mole d'acide cyanhydrique à une température voisine de 20°C. Après 10 minutes de réaction, on introduit 5 moles d'ammoniac liquide. On chauffe à 60°C pendant 30 minutes sous une pression de 11,5 bars tout en agitant fortement. Par détente, on élimine l'ammoniac en excès. Le mélange réactionnel contient environ 0,8 mole d'ammoniac résiduel par mole d'aminonitrile formé. Après refroidissement à une température voisine de 10°C, on introduit 350 g d'une solution aqueuse contenant 0,2 mole d'acétone et 0,2 mole de soude. La température s'élève à 20–25°C. On chauffe à 30°C pendant 1 heure. L'acétone et l'ammoniac en excès sont éliminés sous pression réduite. On ajoute alors 68 g de lessive de soude à 50% (0,85 mole) puis on chauffe à 180°C pendant 5 minutes. La pression est de 19 bars. On décompresse jusqu'à la pression atmosphérique; la température descend au voisinage de 95°C. Après refroidissement à une température voisine de 20°C, on obtient 333 g d'une solution jaune contenant 43,40% en poids de méthionine et 0,055% en poids d'ammoniac.

Le rendement en méthionine est de 37% par rapport à l'AMMP mis en œuvre.

La teneur en ammoniac résiduel peut être diminuée en concentrant sous pression réduite.

Dans ces conditions, on obtient une solution dont les caractéristiques sont les suivantes:
– méthionine 43,7% en poids (0,293 mole/100 g)
– Na 7,08% (0,308 atome-gramme/100 g)
– ammoniac 0,013%
– viscosité 10 centistoke à 30°C
– densité 1,195
– point de cristallisation –23°C
   Par chromatographie en couche mince, les produits suivants sont dosés:
– dicétopipérazine inférieur à 0,01%
– hydantoïne inférieur à 0,01%
– acide hydantoïque inférieur à 0,01%
– uréido butyramide inférieur à 0,01%
– acide diamino-2,4 butyrique 0,5 à 0,6%
– amino-3 pyrrolidone inférieur ou égal à 0,01%
– acide hexahydropyrimidine carboxylique inférieur ou égal à 0,02%
– amide de la méthionine 0,02%
– L,L-dipeptide 0,1%
– D,L-dipeptide 0,1%

## EXEMPLE 2

Dans un réacteur de 500 cm³ permettant de travailler sous pression on introduit 50 g d'eau, 88,8 g de méthylmercapto-4 amino-2 butyramide (0,60 mole) et 50,4 g de lessive de soude à 50% (0,63 mole). On complète le mélange par addition d'eau de façon à obtenir un mélange réactionnel pesant 220 g. On chauffe à 120°C en 10 minutes puis on maintient à cette température pendant 20 minutes tout en agitant. On élimine l'ammoniac formé par détente. La température descend au voisinage de 95°C. Après refroidissement à une température voisine de 20°C, on obtient 205 g d'une solution jaune clair contenant 43,2% en poids de méthionine.

Le rendement est voisin de 99%.

L'efficacité biologique des solutions de méthioninate alcalin obtenues selon le procédé de la présente invention est comparable à celle de la méthionine utilisée habituellement sous forme solide comme il ressort des essais montrant l'influence de ces produits sur la croissance des poulets.

L'essai est réalisé sur des poulets mâles pendant la période 8–28 jours. Les animaux reçoivent l'un des dix régimes expérimentaux issus d'un même régime de base contenant 0,54% d'acide aminés soufrés dont la composition est la suivante:
– maïs 31%
– amidon de maïs 28%
– tourteaux de soja 24%
– gélatine 10%
– suif 3%
– sels minéraux et vitamines 4%
– L-lysine, chlorhydrate 0,04%
– L-tryptophane 0,04%
– D,L-isolucine 0,05%
– D,L-leucine 0,08%
– protéines brutes 23,2%
– lysine 1,18%
– méthionine 0,30%
– méthionine + cystéine 0,54%
– énergie métabolisable 3190 kcal/kg
   Les aliments expérimentaux varient uniquement par le taux d'addition de méthionine (0; 0,05; 0,10 et 0,25%) et aussi par la source de cette supplémentation (méthionine solide contenant 99% de méthionine base (solution de méthioninate de sodium selon l'exemple 1 titrant 43,7% de méthionine base).

Chaque lot est constitué de 12 répétitions de 6 poussins par cage.

Les résultats obtenus sont rassemblés dans le tableau I.

## Tableau I

| Source de méthionine | Taux de supplé-mentation % base | Consommation kg/animal | Gain de poids g/animal | Indice de consommation |
|---|---|---|---|---|
| Régime de base | 0 | 1,160 | 609 | 1,91 |
| D,L-méthionine | 0,047 | 1,179 | 627 | 1,88 |
| D,L-méthioninate de sodium selon l'exemple 1 | 0,064 | 1,228 | 664 | 1,85 |
| D,G-méthionin solide | 0,097 | 1,237 | 684 | 1,81 |
| D,L-métioninate de sodium selon l'exemple 1 | 0,102 | 1,234 | 694 | 1,78 |
| D,L-méthionine solide | 0,235 | 1,251 | 740 | 1,69 |
| D,L-méthioninate de sodium selon l'exemple 1 | 0,228 | 1,254 | 751 | 1,67 |

Dans le tableau II sont rassemblés les résultats montrant la variation du gain de poids en fonction de la source et du niveau de méthionine consommée.

## Tableau II

| Equation de dégression Y + ax + b | Nombre de points par droite | Coefficient corrélation | Efficacité moléculaire |
|---|---|---|---|
| D,L-méthionin solide $Y = 0,083 x + 586$ | 36 | 0,74 | 100 |
| D,L-méthioninate de sodium selon l'exemple 1 $Y = 0,086 x + 591$ | 36 | 0,78 | 104 |

La comparaison des coefficients de régression montre que l'efficacité biologique est la même pour les deux produits considérés.

## Revendications

1. Procédé de préparation d'une solution de méthioninate alcalin exempte de sels minéraux, directement utilisable comme additif pour l'alimentation des animaux d'élevage caractérisé en ce que l'on hydrolyse une quantité suffisante de méthylmercapto-4 amino-2 butyramide au moyen d'un hydroxyde de métal alcalin, élimine l'ammoniac formé et obtient, éventuellement après concentration, une solution de méthioninate alcalin dont la concentration exprimée en équivalent méthionine est comprise entre 30 et 60% en poids.

2. Procédé selon la revendication 1 caractérisé en ce que l'hydrolyse de l'amide est effectuée par une quantité d'hydroxyde alcalin comprise entre 0,9 et 1,1 mole par mole.

3. Procédé selon la revendication 2 caractérisé en ce que l'hydroxyde alcalin est la soude ou la potasse.

4. Procédé selon la revendication 1 caractérisé en ce que l'on effectue l'hydrolyse sous pression à une température comprise entre 100 et 180°C.

5. Procédé selon la revendication 1 caractérisé en ce que l'amide mis en œuvre est obtenu par hydrolyse catalytique du méthylmercapto-4 amino-2 butyronitrile en présence d'ions hydroxyde et d'un composé carboxylé libre ou greffé sur un polymère.

6. Procédé selon la revendication 1 caractérisé en ce que la concentration initiale en méthylmercapto-4 amino-2 butyramide est fixée de façon que la teneur en méthionine de la solution de méthioninate alcalin soit comprise entre 30 et 60%, et de préférence entre 40 et 45% en poids.

7. Solution de méthioninate alcalin qui est exempte de sels minéraux, dont la teneur en méthionine est comprise entre 30 et 60% et qui est directement utilisable comme additif pour l'alimentation des animaux d'élevage lorsqu'elle est obtenue selon le procédé de l'une des revendications 1 à 6.

8. Utilisation d'une solution de méthioninate alcalin selon la revendication 7 comme additif pour l'alimentation des animaux d'élevage.

**Patentansprüche**

1. Verfahren zur Herstellung einer von anorganischen Salzen freien, direkt als Zusatzstoff für Futtermittel für Zuchttiere verwendbaren Lösung von Alkalimethioninat, dadurch gekennzeichnet, daß man eine ausreichende Menge an 4-Methylmercapto-2-amino-butyramid mittels eines Alkalimetallhydroxids hydrolysiert, gebildetes Ammoniak entfernt und, gegebenenfalls nach Einengen, eine Lösung von Alkalimethioninat erhält, deren Konzentration, ausgedrückt in Methioninäquivalent, zwischen 30 und 60 Gew.-% liegt.

2. Verfahren nach Anspruch 1, dadurch gekennzeichnet, daß die Hydrolyse des Amids mit einer Menge an Alkalihydroxid zwischen 0,9 und 1,1 mol pro mol ausgeführt wird.

3. Verfahren nach Anspruch 2, dadurch gekennzeichnet, daß das Alkalihydroxid Natrium- oder Kaliumhydroxid ist.

4. Verfahren nach Anspruch 1, dadurch gekennzeichnet, daß man die Hydrolyse unter Druck bei einer Temperatur zwischen 100 und 180°C ausführt.

5. Verfahren nach Anspruch 1, dadurch gekennzeichnet, daß das verwendete Amid erhalten wird durch katalytische Hydrolyse von 4-Methylmercapto-2-amino-butyronitril in Gegenwart von Hydroxidionen und einer freien oder auf ein Polymer gepfropften Carboxylverbindung.

6. Verfahren nach Anspruch 1, dadurch gekennzeichnet, daß die Ausgangskonzentration an 4-Methylmercapto-2-amino-butyramid derart festgelegt ist, daß der Gehalt der Alkalimethioninatlösung an Methionin zwischen 30 und 60%, vorzugsweise zwischen 40 und 45 Gew.-% liegt.

7. Von anorganischen Salzen freie Lösung von Alkalimethioninat, deren Methioningehalt zwischen 30 und 60% liegt und die direkt als Zusatzstoff für Futtermittel für Zuchttiere verwendbar ist, erhalten nach dem Verfahren nach einem der Ansprüche 1 bis 6.

8. Verwendung einer Alkalimethioninatlösung nach Anspruch 7 als Zusatzstoff für Futtermittel für Zuchttiere.

**Claims**

1. Process for preparing a solution of alkali metal methioninate free from inorganic salts, which solution can be directly used as an additive for feeding livestock, characterized in that a sufficient amount of 4-methylmercapto-2-aminobutyramide is hydrolysed by means of an alkali metal hydroxide, the ammonia formed is removed and a solution of alkali metal methioninate, the concentration of which, expressed in equivalents of methionine, is between 30 and 60% by weight, is obtained, where appropriate after concentration.

2. Process according to Claim 1, characterized in that the hydrolysis of the amide is performed with an amount of alkali metal hydroxide between 0.9 and 1.1 mole per mole.

3. Process according to Claim 2, characterized in that the alkali metal hydroxide is sodium hydroxide or potassium hydroxide.

4. Process according to Claim 1, characterized in that the hydrolysis is performed under pressure at a temperature of between 100 and 180°C.

5. Process according to Claim 1, characterized in that the amide introduced is obtained by catalytic hydrolysis of 4-methylmercapto-2-aminobutyronitrile in the presence of hydroxide ions and a free carboxyl compound or a carboxyl compound grafted onto a polymer.

6. Process according to Claim 1, characterized in that the initial concentration of 4-methylmercapto-2-aminobutyramide is fixed in such a way that the methionine content of the solution of alkali metal methioninate is between 30 and 60%, and preferably between 40 and 45%, by weight.

7. Solution of alkali metal methioninate, the solution being free from inorganic salts and having a methionine content of between 30 and 60%, which solution can be used directly as an additive for feeding livestock when it is obtained according to the process of one of Claims 1 to 6.

8. Use of a solution of alkali metal methioninate according to Claim 7 as an additive for feeding livestock.